# EUROPÄISCHE PATENTSCHRIFT

(11) **EP 0 831 087 B1**
(45) Veröffentlichungstag und Bekanntmachung des Hinweises auf die Patenterteilung: **21.06.2000**
(21) Anmeldenummer: 97116023.9
(22) Anmeldetag: 15.09.1997
(51) Int. Cl.: C07D 211/20, C07D 211/16, C07D 211/82

(54) **Verfahren zur Herstellung von 1-Acyl-4-arylpiperidinen**
Process for the preparation of 1-acyl-4-arylpiperidines
Procédé pour la préparation de 1-acyl-4-arylpiperidines

(30) Priorität: 18.09.1996 CH 228096
(43) Veröffentlichungstag der Anmeldung: 25.03.1998
(73) Patentinhaber: LONZA AG, CH-3945 Gampel/Wallis (CH)
(72) Erfinder: Brieden, Walter, Dr., 3902 Glis (Kanton Wallis) (CH); Loetscher, Didier, 3953 Leuk-Stadt (Kanton Wallis) (CH); Naughton, Andrew, Dr., 3930 Visp (Kanton Wallis) (CH)
(74) Vertreter: Winter, Brandl, Fürniss, Hübner, Röss, Kaiser, Polte Partnerschaft

(56) Entgegenhaltungen:
- EP-A- 0 219 934
- EP-A- 0 630 887
- LISE-LOTT GUNDERSEN ET. AL.: "Aryl- and Alkynyltri-isopropoxytitanium Reagents in Regioselective Carbon-Carbon Bond Formation in Azines." TETRAHEDRON, Bd. 48, Nr. 27, 1992, OXFORD, GB, Seiten 5647-56, XP002051057
- U. ROSENTRETTER ET. AL.: "Stereoselective Synthesis of all-trans Isomers of 1,2,3,4-Tetrahydropyridines and Piperidines form Hantzsch Type 1,4-Dihydropyridines." SYNTHESIS, Nr. 2, Februar 1985, Seiten 210-2, XP002051058

## Beschreibung

Die Erfindung betrifft ein Verfahren zur Herstellung von 1-Acyl-4-arylpiperidinen der allgemeinen Formel 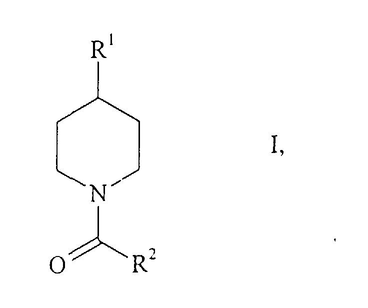 worin R¹ eine gegebenenfalls mit einer oder mehreren C₁₋₆-Alkylgruppen, C₁₋₆-Alkoxygruppen oder C₁₋₆-Alkylthiogruppen (C₁₋₆-Alkylsulfanylgruppen) oder einem oder mehreren Fluoratomen substituierte Arylgruppe und R² eine C₁₋₆-Alkylgruppe, eine C₁₋₆-Alkoxygruppe oder eine gegebenenfalls am Phenylrest mit einem oder mehreren der vorstehend genannten Substituenten substituierte Benzyloxygruppe bedeutet. Sie betrifft weiterhin neue 1-Acyl-4-aryl-1,4-dihydropyridine als Zwischenprodukte im erfindungsgemässen Verfahren.

Verbindungen dieser Struktur, insbesondere solche, bei denen R² mit der benachbarten Carbonylgruppe eine Aminoschutzgruppe wie beispielsweise Benzyloxycarbonyl bildet, sind Zwischenprodukte in der Synthese von Neurokininrezeptor-Antagonisten (EP-A 0 630 887, WO-A 95/16682).

Hier und im folgenden sind unter C₁₋₆-Alkylgruppen jeweils lineare und verzweigte, primäre, sekundäre oder tertiäre Alkylgruppen mit 1-6 Kohlenstoffatomen zu verstehen, also beispielsweise Methyl, Ethyl, Propyl, Isopropyl, Butyl, Isobutyl, sec-Butyl, tert-Butyl, Pentyl, Isopentyl, Neopentyl, Hexyl und so weiter. Entsprechend sind unter C₁₋₆-Alkoxygruppen und C₁₋₆-Alkylthiogruppen die aus den vorstehend genannten C₁₋₆-Alkylgruppen und Sauerstoff bzw. Schwefel zusammengesetzten Ether- bzw Thioethergruppen zu verstehen.

Unter Arylgruppen sind mono-, bi- und polycyclische carbo- oder heterocyclische aromatische Reste zu verstehen, also beispielsweise Phenyl, Naphthyl, Biphenylyl, Anthracenyl, Furyl, Thiophenyl usw

Bisher bekannte Synthesen von Verbindungen der Struktur I gehen von 1-Acyl-4-piperidonen aus, die mit Arylmagnesiumhalogeniden zu den entsprechenden 1-Acyl-4-aryl-4-hydroxypiperidinen umgesetzt werden. Diese können entweder mit Triethylsilan direkt zu den gewünschten Produkten reduziert oder durch Elimination von Wasser zu den entsprechenden 1,2,3,6-Tetrahydropyridinen und anschliessende Hydrierung der so gebildeten Doppelbindung in diese übergeführt werden.. Beide Verfahren haben den Nachteil, dass sie ein teures Ausgangsmaterial (1-Acyl-4-piperidon) erfordern, das erstgenannte Verfahren benötigt ausserdem noch ein teures Reagenz (Triethylsilan).

Die EP-A-0 219 934 beschreibt ein Verfahren zur Herstellung von 1-Alkoxy-4-aryl-1,4-dihydropyridin-3-carbonsäureestern, bei dem ein Arylmagnesiumhalogenid in Gegenwart eines Acylhalogenids und eines Kupfersalzes mit einem Nikotinat umgesetzt wird. Das erhaltene Dihydropyridin wird dann zum Pyridin aromatisiert. Ein Verfahren zur Herstellung eines 4-Phenylpyridins durch Reaktion einer Organotitan-Verbindung mit 1-lsobutyloxycarbonylpyridin wird von L.L. Gunderson et al. (Tetrahedron 48 (1992) 5647-56) beschrieben

U. Rosentreter (Synthesis 2 (1985) 210-212) offenbart die Hydrierung von 1,4-Dihydropyridinen, wie sie nach der Hantzsch-Synthese erhältlich sind, zu Piperidinen. Die Hydrierung erfolgt mit Triethylsilan in Gegenwart von Trifluoressigsäure.

Aufgabe der vorliegenden Erfindung war es daher, ein alternatives Verfahren zur Verfügung zu stellen, das von billigeren Rohstoffen ausgeht und für die Durchführung in technischem Massstab geeignet ist.

Erfindungsgemäss wird diese Aufgabe durch das Verfahren nach Patentanspruch 1 und die neuen Zwischenprodukte nach Patentanspruch 11 gelöst.

Es wurde gefunden, dass Arylmagnesiumhalogenide der allgemeinen Formel

R¹-MgX¹ II,

worin R¹ die oben genannte Bedeutung hat und X¹ Chlor, Brom oder Iod ist, mit den aus Pyridin und Acylhalogeniden der allgemeinen Formel

R²-COX² III,

worin R² die oben genannte Bedeutung hat und X² Chlor oder Brom ist, erhältlichen 1-Acylpyridiniumhalogeniden in Gegenwart einer Kupferverbindung zu 1-Acyl-4-aryl-1,4-dihydropyridinen der allgemeinen Formel 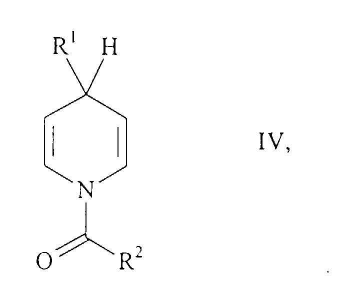 worin R¹ und R² die oben genannten Bedeutungen haben, umgesetzt werden können und sich diese unter homogener Katalyse zu den gewünschten 1-Acyl-4-arylpiperidinen hydrieren lassen.

Als Arylgruppe R¹ im Arylmagnesiumhalogenid II wird vorzugsweise eine Phenylgruppe eingesetzt, die gegebenenfalls mit einer oder mehreren C₁₋₆-Alkylgruppen, C₁₋₆-Alkoxygruppen, Benzyloxygruppen oder C₁₋₆-Alkylthiogruppen oder einem oder mehreren Fluoratomen substituiert sein kann. Besonders bevorzugt ist eine 2-(Methylthio)phenylgruppe.

Als Halogen im Arylmagnesiumhalogenid II wird vorzugsweise Brom eingesetzt.

Die Arylmagnesiumhalogenide können auf die übliche Weise aus Magnesiummetall und den entsprechenden Arylhalogeniden hergestellt werden. Letztere sind bekannte Verbindungen und kommerziell erhältlich oder nach bekannten Verfahren leicht zugänglich.

Als 1-Acyl-Substituent wird vorzugsweise die Benzyloxycarbonylgruppe eingesetzt. R² ist in diesem Fall eine Benzyloxygruppe.

Reaktionspartner des Arylmagnesiumhalogenids ist das aus dem Acylhalogenid III und Pyridin erhältliche 1-Acylpyridiniumhalogenid der allgemeinen Formel 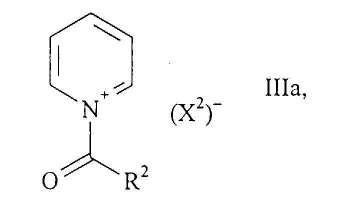 in welchem R² und X² die oben genannten Bedeutungen haben.

Vorzugsweise ist X² Chlor.

Das 1-Acylpyridiniumhalogenid IIIa kann sowohl in einem Eintopfverfahren bzw. in situ hergestellt als auch in einem separaten Schritt hergestellt und in Substanz isoliert werden. Es liegt auch im Rahmen der vorliegenden Erfindung, ein auf anderem Weg als durch direkte Umsetzung von Pyridin mit dem Acylhalogenid III hergestelltes 1-Acylpyridiniumhalogenid IIIa einzusetzen.
Besonders bevorzugt sind die Ausführungsformen, in denen das 1-Acylpyridiniumhalogenid nicht isoliert, sondern aus Acylhalogenid III und Pyridin in einem Eintopfverfahren bzw. in situ gebildet wird. Hierzu kann das Pyridin vorgelegt und zuerst mit dem Acylhalogenid III im wesentlichen vollständig zum 1-Acylpyridiniumhalogenid IIIa umgesetzt werden, zu welchem dann langsam das Arylmagnesiumhalogenid gegeben wird ("Eintopfverfahren"). In einer anderen Ausführungsform wird das Pyridin zusammen mit dem Arylmagnesiumhalogenid II und der Kupferverbindung vorgelegt und das Acylhalogenid III wird langsam zugegeben. Hierbei reagiert das Acylhalogenid zunächst mit dem Pyridin und das so in situ gebildete 1-Acylpyridiniumhalogenid setzt sich unmittelbar danach mit dem Arylmagnesiumhalogenid um.

Die Bildung des 1-Acylpyridiumhalogenids IIIa erfolgt vorteilhaft bei einer Temperatur von -80 bis +40 °C, vorzugsweise bei -40 bis -10 °C. Als Lösungsmittel wird zweckmässig ein aprotisches Lösungsmittel eingesetzt, das nicht mit Grignard-Verbindungen reagiert. Vorzugsweise werden Ether wie beispielsweise Tetrahydrofuran als Lösungsmittel verwendet.

Die Umsetzung mit dem Arylmagnesiumhalogenid II wird vorteilhaft unter den gleichen Bedingungen wie die Bildung des 1-Acylpyridiniumhalogenids IIIa durchgeführt.

Die Kupferverbindung kann sowohl vor der Bildung des 1-Acylpyridiniumhalogenids IIIa als auch vor seiner Umsetzung mit dem Arylmagnesiumhalogenid II zugegeben werden. Der Zusatz der Kupferverbindung bewirkt die regioselektive Reaktion in Position 4 des Pyridinrings. Ohne Kupferzusatz wird im allgemeinen ein Gemisch aus den Produkten der Addition in Position 2 (bzw. 6) und 4 erhalten.
Besonders bevorzugt als Kupferverbindung ist Kupfer(I)iodid.

Als homogene Katalysatoren für die Hydrierung der 1-Acyl-4-aryl-1,4-dihydropyridine IV werden vorzugsweise Platinmetall-Phosphin-Komplexe eingesetzt. Als Platinmetalle kommen beispielsweise Ruthenium, Rhodium, Palladium, Iridium oder Platin in Frage. Besonders bevorzugt sind Rhodium-Phosphin-Komplexe, insbesondere das Tris-triphenylphosphinrhodium(I)chlorid. Hiermit lassen sich überraschenderweise auch 1-Acyl-4-aryl-1,4-dihydropyridine mit schwefelhaltigen Substituenten und O-Benzyl-Gruppen wie beispielsweise das 1-Benzyloxycarbonyl-4-[2-(methylthio)phenyl]-1,4-dihydropyridin in guter Ausbeute hydrieren, ohne dass eine Katalysatorvergiftung oder eine Hydrogenolyse der Benzylgruppe stattfindet.
Die Hydrierung wird vorteilhaft bei einer Temperatur von Raumtemperatur bis 100 °C durchgeführt, besonders bevorzugt bei 60-80 °C. Der Wasserstoffdruck beträgt vorteilhaft 1-100 bar, besonders bevorzugt ist der Bereich von 2-50 bar. Als Lösungsmittel für die Hydrierung eignen sich die für katalytische Hydrierungen üblichen Lösungsmittel, beispielsweise niedere Alkohole wie Methanol oder Ethanol, Ester wie Ethylacetat, oder Kohlenwasserstoffe wie beispielsweise Toluol.

Vorteilhafte neue 1-Acyl-4-aryl-1,4-dihydropyridine als Zwischenprodukte des erfindungsgemässen Verfahrens sind die 1-Benzyloxycarbonyl-4-aryl-1,4-dihydropyridine der allgemeinen Formel 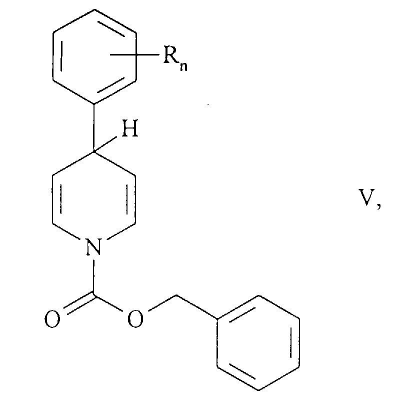 worin n eine ganze Zahl von 0 bis 5 ist und jeder Rest R unabhängig von den anderen ausgewählt ist aus der Gruppe bestehend aus Fluor, C₁₋₆-Alkyl, C₁₋₆-Alkoxy, Benzyloxy und C₁₋₆-Alkylthio.
Besonders bevorzugt ist das 1-Benzyloxycarbonyl-4-[2-(methylthio)phenyl]-1,4-dihydropyridin der Formel

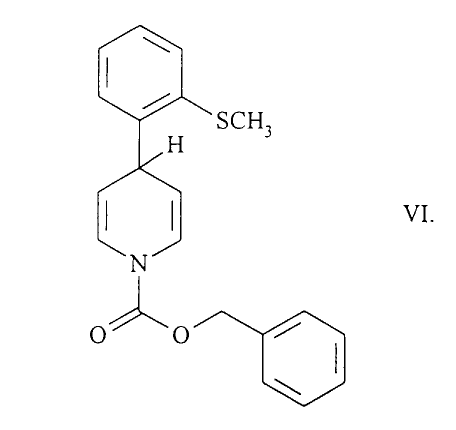

Die 1-Benzyloxycarbonyl-4-aryl-1,4-dihydropyridine V werden vorzugsweise dadurch hergestellt, dass ein Phenylmagnesiumhalogenid der allgemeinen Formel 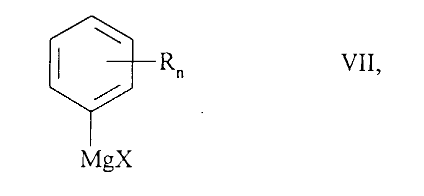 worin X Chlor, Brom oder Iod ist und n und R die oben genannten Bedeutungen haben, in Gegenwart von Kupfer(I)iodid mit aus Pyridin und Chlorameisensäurebenzylester gebildetem 1-Benzyloxycarbonylpyridiniumchlorid umgesetzt wird.

Die nachfolgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### 1-Benzyloxycarbonyl-4-[2-(methylthio)phenyl]-1,4-dihydropyridin (IV, R¹ = 2-(CH₃S)C₆H₄, R² = OCH₂C₆H₅)

Unter Argon wurden in 100 ml Tetrahydrofuran (über Molekularsieb getrocknet) 2,9 g (118 mmol) Magnesiumspäne vorgelegt. Bei Raumtemperatur wurden ca. 5 g 2-Brom-thioanisol zugetropft und das Gemisch auf 50 °C erwärmt. Nach dem Anspringen der Grignard-Reaktion erwärmte sich das Gemisch weiter bis zum Rückfluss. Der Rest der Gesamtmenge von 20,0 g (98,5 mmol) 2-Brom-thioanisol wurde so zugetropft, dass das Reaktionsgemisch weiterhin am Rückfluss siedete. Anschliessend liess man die so erhaltene Lösung der Grignard-Verbindung 2-(Methylthio)phenylmagnesiumbromid langsam auf Raumtemperatur abkühlen. In einem zweiten Kolben wurden unter Argon 250 ml getrocknetes Tetrahydrofuran vorgelegt, mit 11,7 g (148 mmol) Pyridin und 0,93 g (4,9 mmol) Kupfer(I)iodid versetzt und auf -30 °C abgekühlt. Bei -30 bis -25 °C wurden 16,8 g (98,5 mmol) Chlorameisensäurebenzylester zugetropft, wobei sich zunächst ein gelbroter Niederschlag und schliesslich eine beige Suspension bildete. Zu dieser wurde bei der gleichen Temperatur die Lösung der Grignard-Verbindung zugetropft; anschliessend wurde die Kühlung entfernt und das Gemisch nach dem Erreichen der Raumtemperatur noch 2 h gerührt. Das Reaktionsgemisch wurde in 120 ml 20%ige wässrige Ammoniumchloridlösung gegossen und heftig gerührt. Dann wurden 500 ml Toluol zugegeben und die Phasen getrennt. Die tiefblaue wässrige Phase wurde dreimal mit je 50 ml Toluol extrahiert, die vereinigten organischen Phasen über Natriumsulfat getrocknet und am Rotationsverdampfer bis zur Trockne eingeengt. Der braune Rückstand wurde zur Entfernung von Lösungsmittelresten noch 1 h unter Hochvakuum gehalten, dann in 500-1000 ml siedendem Methanol aufgelöst und heiss filtriert. Das Filtrat wurde langsam auf Raumtemperatur und schliesslich auf +2 °C abgekühlt, wobei das Produkt als voluminöser gelblich-weisser Niederschlag ausfiel. Dieser wurde abfiltriert und bei 35 °C im Vakuum getrocknet.
Ausbeute: 20,8 g (62,7 %) gelblich-weisser Festkörper
Schmp.: 90,1-90,9 °C
¹H NMR (CDCl₃): δ = 2,46 (s, 3H); 4,65 (m, 1H), 4,95 (br. d, 2H); 5,24 (s, 2H); 6,94 (br. d, 2H); 7,17-7,41 (m, 9H).
¹³C NMR (CDCl₃): δ = 16,17; 35,43; 68,21; 108,67; 109,15; 122,49; 122,90; 125,80-129,40 (7 Signale); 135,72; 135,79; 143,35; 151,36.
IR (Film): 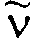 = 1720,0 cm⁻¹ (C=O); 1690,8 (C=C).
MS: m/z = 337 (M⁺); 322; 292; 278; 247; 246; 214; 202; 186; 155; 115; 91 (100%); 65.

### Beispiel 2

### 1-Benzyloxycarbonyl-4-[2-(methylthio)phenyl]piperidin (I, R¹ = 2-(CH₃S)C₆H₄, R² = OCH₂C₆H₅)

In einem Autoklaven wurde eine Lösung von 0,50 g (1,48 mmol) 1-Benzyloxycarbonyl-4-[2-(methylthio)phenyl]-1,4-dihydropyridin (hergestellt nach Beispiel 1) in 30 ml entgastem absolutem Ethanol mit 0,137 g (0,148 mmol) Tris-triphenylphosphin-rhodium(I)-chlorid versetzt. Das Gemisch wurde unter 45 bar Wasserstoffdruck gesetzt, auf 70 °C erwärmt und über Nacht unter diesen Bedingungen gerührt. Nach dem Abkühlen auf Raumtemperatur und dem Ablassen des Wasserstoffs wurde das dunkelbraune Reaktionsgemisch über Celite® filtriert und das Ethanol am Rotationsverdampfer abdestilliert. Der braune ölige Rückstand wurde mit Ethylacetat/Hexan (1:10) an Kieselgel 60 chromatographiert.
Ausbeute: 0,386 g (76,3%) farbloses Öl, das beim Stehenlassen teilweise erstarrt.
Zur Reinigung wurde eine Probe aus wenig Ethanol in der Kälte (0 °C) kristallisiert und bei 40 °C im Vakuum getrocknet.
Schmp.: 74,9-75,9 °C
- ¹H NMR (CDCl₃): δ =: 1,50-1,60 (m, 2H); 1,80-1,90 (m, 2H); 2,45 (s, 3H); 2,95 (br. t, 2H); 3,18 (tt, 1H); 4,27 (br. s, 2H); 5,17 (s, 2H); 7,15-7,40 (m, 9H).
- ¹³C NMR (CDCl₃): δ =: 16,24; 32,19; 38,81; 44,82; 67,09; 125,50-128,53 (7 Signale); 136,72; 137,04; 143,30; 155,40.
- MS: m/z =: 341 (M⁺); 296; 250; 206; 177; 135; 91; 56.

## Patentansprüche

1. Verfahren zur Herstellung von 1-Acyl-4-arylpiperidinen der allgemeinen Formel 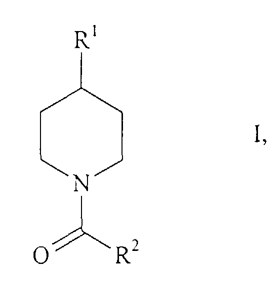 worin R¹ eine gegebenenfalls mit einer oder mehreren C₁₋₆-Alkylgruppen, C₁₋₆-Alkoxygruppen, Benzyloxygruppen oder C₁₋₆-Alkylthiogruppen und/oder einem oder mehreren Fluoratomen substituierte Arylgruppe und R² eine C₁₋₆-Alkylgruppe, eine C₁₋₆-Alkoxygruppe, eine gegebenenfalls mit einer oder mehreren C₁₋₆-Alkylgruppen, C₁₋₆-Alkoxygruppen, Benzyloxygruppen oder C₁₋₆-Alkylthiogruppen und/oder einem oder mehreren Fluoratomen substituierte Arylgruppe, Arylalkylgruppe oder eine gegebenenfalls am Phenylrest mit einer oder mehreren C₁₋₆-Alkylgruppen, C₁₋₆-Alkoxygruppen, Benzyloxygruppen oder C₁₋₆-Alkylthiogruppen und/oder einem oder mehreren Fluoratomen substituierte Benzyloxygruppe bedeutet, dadurch gekennzeichnet, dass in einer ersten Stufe ein Arylmagnesiumhalogenid der allgemeinen Formel
R¹-MgX¹ II,
worin X¹ Chlor, Brom oder Iod ist und R¹ die oben genannte Bedeutung hat, mit einem aus Pyridin und einem Acylhalogenid der allgemeinen Formel
R²-COX² III,
worin X² Chlor oder Brom ist und R² die oben genannte Bedeutung hat, erhältlichen 1-Acylpyridiniumhalogenid in Gegenwart einer Kupferverbindung zum 1-Acyl-4-aryl-1,4-dihydropyridin der allgemeinen Formel 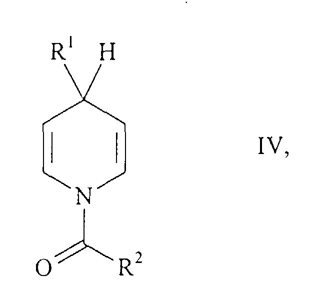 worin R¹ und R² die oben genannten Bedeutungen haben, umgesetzt und in einer zweiten Stufe unter homogener Katalyse hydriert wird.

2. Verfahren nach Anspruch 1, dadurch gekennzeichnet, dass R¹ eine gegebenenfalls mit einer oder mehreren C₁₋₆-Alkylgruppen, C₁₋₆-Alkoxygruppen, Benzyloxygruppen oder C₁₋₆-Alkylthiogruppen oder einem oder mehreren Fluoratomen substituierte Phenylgruppe ist.

3. Verfahren nach Anspruch 2, dadurch gekennzeichnet, dass R¹ eine 2-(Methylthio)-phenylgruppe ist.

4. Verfahren nach einem der Ansprüche 1 bis 3, dadurch gekennzeichnet, dass X¹ Brom ist.

5. Verfahren nach einem der Ansprüche 1 bis 4, dadurch gekennzeichnet, dass R² eine Benzyloxygruppe ist.

6. Verfahren nach einem der Ansprüche 1 bis 5, dadurch gekennzeichnet, dass X² Chlor ist.

7. Verfahren nach einem der Ansprüche 1 bis 6, dadurch gekennzeichnet, dass das 1-Acylpyridiniumhalogenid nicht isoliert wird.

8. Verfahren nach einem der Ansprüche 1 bis 7, dadurch gekennzeichnet, dass als Kupferverbindung Kupfer(I)iodid eingesetzt wird.

9. Verfahren nach einem der Ansprüche 1 bis 8, dadurch gekennzeichnet, dass die homogen katalysierte Hydrierung mit einem Rhodium-Phosphin-Komplex als Katalysator durchgeführt wird.

10. Verfahren nach Anspruch 9, dadurch gekennzeichnet, dass als Rhodium-Phosphin-Komplex Tris-triphenylphosphinrhodium(I)chlorid eingesetzt wird.

11. 1-Benzyloxycarbonyl-4-aryl-1,4-dihydropyridine der allgemeinen Formel 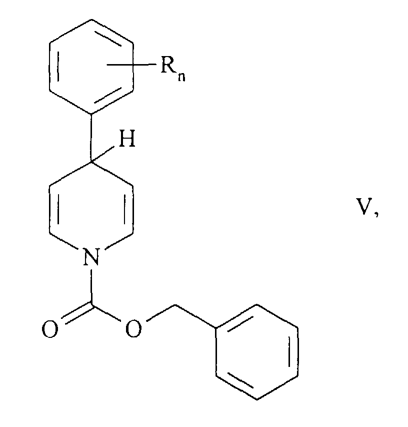 worin n eine ganze Zahl von 0 bis 5 ist und jeder Rest R unabhängig von den anderen ausgewählt ist aus der Gruppe bestehend aus Fluor, C₁₋₆-Alkyl, C₁₋₆-Alkoxy, Benzyloxy und C₁₋₆-Alkylthio.

12. 1-Benzyloxycarbonyl-4-[2-(methylthio)phenyl]-1,4-dihydropyridin der Formel

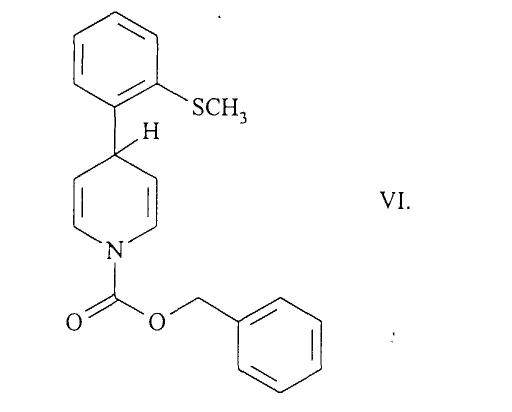

13. Verfahren zur Herstellung von 1-Benzyloxycarbonyl-4-aryl-1,4-dihydropyridinen der allgemeinen Formel V gemäss Anspruch 11, dadurch gekennzeichnet, dass ein Phenylmagnesiumhalogenid der allgemeinen Formel 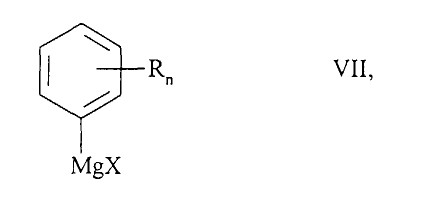 worin X Chlor, Brom oder Iod ist und n und R die in Anspruch 11 genannten Bedeutungen haben, in Gegenwart von Kupfer(I)iodid mit aus Pyridin und Chlorameisensäurebenzylester gebildetem 1-Benzyloxycarbonylpyridiniumchlorid umgesetzt wird.

## Revendications

1. Procédé de préparation de 1-acyl-4-arylpipéridines de formule générale 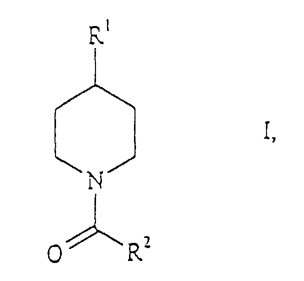 dans laquelle R¹ représente un groupe aryle éventuellement substitué par un ou plusieurs groupes alkyle en C₁-C₆, alcoxy en C₁-C₆, benzyloxy ou alkylthio en C₁-C₆ et/ou par un ou plusieurs atomes de fluor, et R² est un groupe alkyle en C₁-C₆, un groupe alcoxy en C₁-C₆, un groupe aryle, arylalkyle éventuellement substitué par un ou plusieurs groupes alkyle en C₁-C₆, alcoxy en C₁-C₆, benzyloxy ou alkylthio en C₁-C₆ et/ou par un ou plusieurs atomes de fluor, ou un groupe benzyloxy éventuellement substitué sur le reste phényle par un ou plusieurs groupes alkyle en C₁-C₆, alcoxy en C₁-C₆, benzyloxy ou alkylthio en C₁-C₆ et/ou par un ou plusieurs atomes de fluor, caractérisé en ce que, dans une première étape, on fait réagir un halogénure d'arylmagnésium de formule générale
R¹-MgX¹ II,
dans laquelle X¹ représente un atome de chlore, de brome ou d'iode et R¹ a la signification donnée ci-dessus, avec un halogénure de 1-acylpyridinium pouvant être obtenu à partir de pyridine et d'un halogénure d'acyle de formule générale
R²-COX² III,
dans laquelle X² est un atome de chlore ou de brome et R² a la signification indiquée ci-dessus, en présence d'un composé du cuivre, pour obtenir la 1-acyl-4-aryl-1,4-dihydropyridine de formule générale 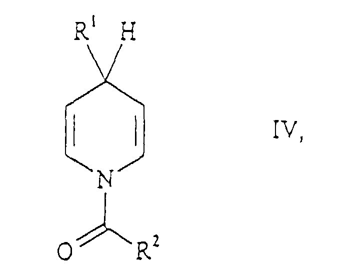 dans laquelle R¹ et R² ont la signification indiquée ci-dessus, et, dans une deuxième étape, on procède à une hydrogénation dans les conditions d'une catalyse homogène.

2. Procédé selon la revendication 1, caractérisé en ce que R¹ est un groupe phényle éventuellement substitué par un ou plusieurs groupes alkyle en C₁-C₆, alcoxy en C₁-C₆, benzyloxy ou alkylthio en C₁-C₆ ou par un ou plusieurs atomes de fluor.

3. Procédé selon la revendication 2, caractérisé en ce que R¹ est un groupe 2-(méthylthio)phényle.

4. Procédé selon l'une des revendications 1 à 3, caractérisé en ce que X¹ est un atome de brome.

5. Procédé selon l'une des revendications 1 à 4, caractérisé en ce que R² est un groupe benzyloxy.

6. Procédé selon l'une des revendications 1 à 5, caractérisé en ce que X² est un atome de chlore.

7. Procédé selon l'une des revendications 1 à 6, caractérisé en ce que l'on n'isole pas l'halogénure de 1-acylpyridinium.

8. Procédé selon l'une des revendications 1 à 7, caractérisé en ce que l'on utilise de l'iodure de cuivre (I) comme composé du cuivre.

9. Procédé selon l'une des revendications 1 à 8, caractérisé en ce que l'on effectue l'hydrogénation à catalyse homogène avec un complexe de rhodium et de phosphine comme catalyseur.

10. Procédé selon la revendication 9, caractérisé en ce que l'on utilise du chlorure de tris-triphénylphosphine-rhodium (I) comme complexe de rhodium et de phosphine.

11. 1-Benzyloxycarbonyl-4-aryl-1,4-dihydropyridines de formule générale 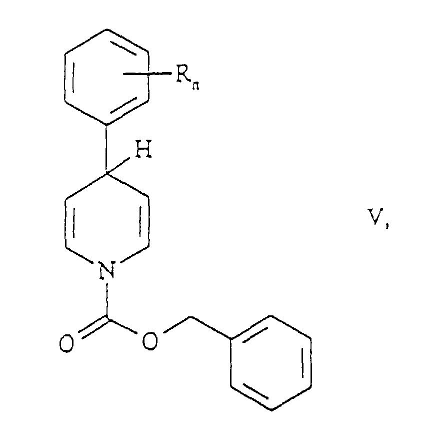 dans laquelle n est un nombre entier de 0 à 5 et chacun des restes R est choisi indépendamment des autres dans le groupe constitué par le fluor et les groupes alkyle en C₁-C₆, alcoxy en C₁-C₆, benzyloxy et alkylthio en C₁-C₆.

12. 1-Benzyloxycarbonyl-4-[2-(méthylthio)phényl]-1,4-dihydropyridine de formule

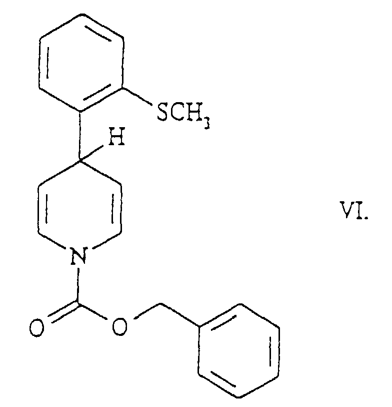

13. Procédé de préparation de 1-benzyloxycarbonyl-4-aryl-1,4-dihydropyridines de formule générale V selon la revendication 11, caractérisé en ce que l'on fait réagir un halogénure de phénylmagnésium de formule générale 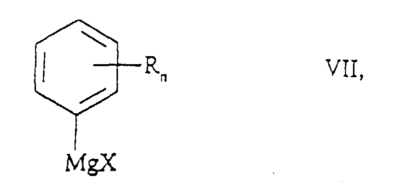 dans laquelle X est un atome de chlore, de brome ou d'iode et n et R ont la signification indiquée dans la revendication 11, en présence d'iodure de cuivre (I), avec du chlorure de 1-benzyloxycarbonylpyridinium formé à partir de pyridine et de chloroformate de benzyle.

## Claims

1. Process for the preparation of 1-acyl-4-arylpiperidines of the general formula 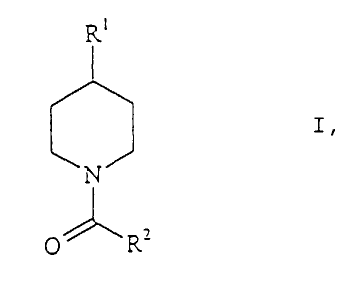 wherein R¹ represents an aryl group optionally substituted by one or more C₁₋₆-alkyl groups, C₁₋₆-alkoxy groups, benzyloxy groups or C₁₋₆-alkylthio groups and/or by one or more fluorine atoms, and R² represents a C₁₋₆-alkyl group, a C₁₋₆-alkoxy group, an aryl group or an arylalkyl group each optionally substituted by one or more C₁₋₆-alkyl groups, C₁₋₆-alkoxy groups, benzyloxy groups or C₁₋₆-alkylthio groups and/or by one or more fluorine atoms, or a benzyloxy group optionally substituted at the phenyl radical by one or more C₁₋₆-alkyl groups, C₁₋₆-alkoxy groups, benzyloxy groups or C₁₋₆-alkylthio groups and/or by one or more fluorine atoms, characterised in that in a first step an arylmagnesium halide of the general formula
R¹-MgX¹ II,
wherein X¹ is chlorine, bromine or iodine and R¹ is as defined above, is reacted in the presence of a copper compound with a 1-acylpyridinium halide obtainable from pyridine and an acyl halide of the general formula
R²-COX² III,
wherein X² is chlorine or bromine and R² is as defined above, to form a 1-acyl-4-aryl-1,4-dihydropyridine of the general formula 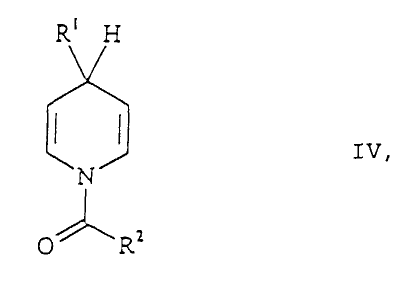 wherein R¹ and R² are as defined above, and in a second step that compound is hydrogenated with homogeneous catalysis.

2. Process according to claim 1, characterised in that R¹ is a phenyl group optionally substituted by one or more C₁₋₆-alkyl groups, C₁₋₆-alkoxy groups, benzyloxy groups or C₁₋₆-alkylthio groups or by one or more fluorine atoms.

3. Process according to claim 2, characterised in that R¹ is a 2-(methylthio)phenyl group.

4. Process according to any one of claims 1 to 3, characterised in that X¹ is bromine.

5. Process according to any one of claims 1 to 4, characterised in that R² is a benzyloxy group.

6. Process according to any one of claims 1 to 5, characterised in that X² is chlorine.

7. Process according to any one of claims 1 to 6, characterised in that the 1-acylpyridinium halide is not isolated.

8. Process according to any one of claims 1 to 7, characterised in that copper(I) iodide is used as the copper compound.

9. Process according to any one of claims 1 to 8, characterised in that the homogeneously catalysed hydrogenation is carried out using a rhodium-phosphine complex as catalyst.

10. Process according to claim 9, characterised in that tris-triphenylphosphinerhodium(I) chloride is used as the rhodium-phosphine complex.

11. 1-Benzyloxycarbonyl-4-aryl-1,4-dihydropyridines of the general formula 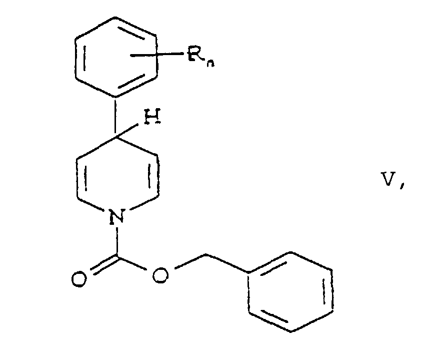 wherein n is an integer from 0 to 5 and each radical R is selected independently of the others from the group consisting of fluorine, C₁₋₆-alkyl, C₁₋₆-alkoxy, benzyloxy and C₁₋₆-alkylthio.

12. 1-Benzyloxycarbonyl-4-[2-(methylthio)phenyl]-1,4-dihydropyridine of the formula

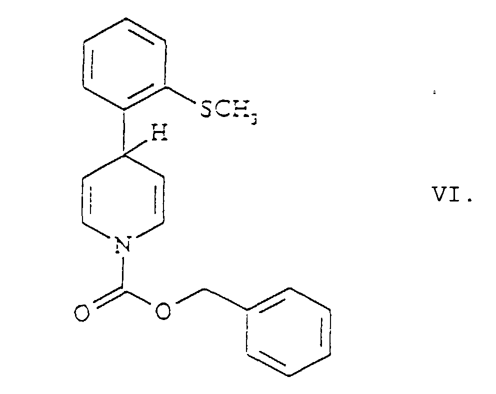

13. Process for the preparation of 1-benzyloxycarbonyl-4-aryl-1,4-dihydropyridines of the general formula V according to claim 11, characterised in that a phenylmagnesium halide of the general formula 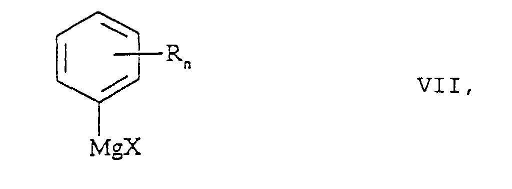 wherein X is chlorine, bromine or iodine and n and R are as defined in claim 11, is reacted in the presence of copper(I) iodide with 1-benzyloxycarbonylpyridinium chloride formed from pyridine and chloroformic acid benzyl ester.
